# EUROPEAN PATENT SPECIFICATION

(11) **EP 1 606 413 B1**
(45) Date of publication and mention of the grant of the patent: **18.10.2006**
(21) Application number: 03816358.0
(22) Date of filing: 28.03.2003
(51) Int. Cl.: C12P 7/64, C12N 1/10

(54) **A METHOD FOR ENHANCING LEVELS OF POLYUNSATURATED FATTY ACIDS IN THRAUSTOCHYTRID PROTISTS**
VERFAHREN ZUR VERBESSERUNG DES STANDS VON MEHRFACH UNGESÄTTIGTEN FETTSÄUREN IN THRAUSTOCHYTRID-PROTISTEN
PROCEDE PERMETTANT D'ACCROITRE LES TAUX D'ACIDES GRAS POLYINSATURES DANS DES PROTISTES DU TYPE THRAUSTOCHYTRIUM

(30) Priority: 21.03.2003 US 393366
(43) Date of publication of application: 21.12.2005
(73) Proprietor: COUNCIL OF SCIENTIFIC AND INDUSTRIAL RESEARCH, New Delhi 110 001 (IN)
(72) Inventor: KUMAR, Seshagiri Raghu, 403 004 Goa (IN); JAIN, Ruchi, 403 004 Goa (IN); KAMATH, Jyothi, 561 229 Bangalore (IN); PRABHU, Surekha, 561 229 Bangalore (IN); SURYANARAYAN, Shrikumar, 561 229 Bangalore (IN)
(74) Representative: Winkler, Andreas Fritz Ernst
(86) International application number: PCT/IN2003/000094
(87) International publication number: WO 2004/083442

(56) References cited:
- US-A- 5 340 594
- US-B1- 6 410 282
- BAJPAI P K ET AL: "OPTIMIZATION OF PRODUCTION OF DOCOSAHEXAENOIC ACID (DNA) BY THRAUSTOCHYTRIUM AUREUM ATCC 34304" JOURNAL OF THE AMERICAN OIL CHEMISTS' SOCIETY, AMERICAN OIL CHEMISTS' SOCIETY. CHAMPAIGN, US, vol. 68, no. 7, July 1991 (1991-07), pages 509-514, XP008023474 ISSN: 0003-021X
- BAJPAI ET AL.: "Eicosapentaenoic acid (EPA) production from microorganisms: a reiew" JOURNAL OF BIOTECHNOLOGY, vol. 30, 1993, pages 161-183, XP002319908 cited in the application
- YOKOCHI T ET AL: "OPTIMIZATION OF DOCOSAHEXAENOIC ACID PRODUCTION BY SCHIZOCHYTRIUM LIMACINUM SR21" APPLIED MICROBIOLOGY AND BIOTECHNOLOGY, SPRINGER VERLAG, BERLIN, DE, vol. 49, 1998, pages 72-76, XP002928890 ISSN: 0175-7598 cited in the application

## Description

### Field of the present Invention

The present invention relates to a method of enhancing the levels of Poly-unsaturated fatty acids (PUFA) by 0.5 to 5 times in protists thraustochytrid protists by storing the cells under cold conditions, said method comprising steps of inoculating the protests into a culture medium, growing the culture for 2 to 5 days at room temperature, harvesting the cells by centrifugation to obtain biomass, storing the biomass at about 10°C for time duration ranging between 12 to 48 hours, and obtaining increased PUFA from the stored biomass.

### Background and prior art references of the present invention

Fatty acids are constituents of lipids, which are required by all living organisms for growth, survival and reproduction. Among the fatty acids, saturated fatty acids are those with a chemical structure in which the carbon atoms are connected to each other only by single bonds and contain no double bonds. Unsaturated fatty acids are those in which one or more of the carbon atoms are connected to each other by double bonds. Polyunsaturated fatty acids, termed as PUFAs hereafter, are those in which more than one such double bonds are found.

Among the PUFAs, two are considered extremely essential in the health of animals and human beings. These are the docosahexaenoic acid and eicosapentaenoic acid, termed DHA and EPA hereafter. The molecular structure of both DHA and EPA is such that the first double bond follows the third carbon atom from the methyl end of the fatty acid structure. Therefore, these are also called omega-3 PUFAs. DHA contains 22 carbon atoms, between which six double bonds are found. EPA contains 20 carbon atoms, between which five double bonds occur.

Both DHA and EPA have been shown to be important for human health and in animal nutrition. In human health, DHA and EPA have been shown to be important in brain development in children, prevention of atherosclerosis, prevention of night blindness, neurological disorders and even for possible prevention of cancer (Bajpai, P. and P. K. Bajpai. 1993. Journal of Biotechnology 30: 161-183; Barclay, W. R. et al. 1994. Journal of Applied Phycology 6: 123-129; Singh, A. and O. P. Ward. 1997. Advances in applied microbiology, 45: 271-312).

These two omega-3 PUFAs have been shown to enhance growth and reproduction in crustacean animals, such as prawns, which are very important as aquaculture animals for human consumption (Harrison, K. E. 1990. Journal of Shellfish Research 9: 1-28). Incorporation of DHA and EPA in human and animal feeds is therefore considered important. DHA and EPA levels of thraustochytrid protists can be enhanced beyond their natural levels by growing the cells in a medium with increased viscosity, and their cells can be of still better use as supplement to human nutrition and as feed for animals compared to presently known processes. Thraustochytrids can be cultivated on a large scale, using well established fermentation techniques. Cells thus obtained can be used as animal feeds, by suitably processing and preserving their cells, such as by spray-drying and freezing. The cell biomass, enhanced in the omega-3 fatty acids can also be harvested and DHA and EPA extracted in a pure form. These may be used to supplement human food that is poor in these essential omega-3 PUFAs.

One major source of EPA and DHA for human consumption is in the form of fish oil. However, fish oil has the disadvantage of an odour, which is disagreeable to many human consumers. Fish containing DHA and EPA are also highly seasonal and variable in their omega-3 PUFA contents. Besides, most of the fish oil is hydrogenated and the omega-3 PUFAs are destroyed. For these reasons, micro-organisms containing EPA and DHA, which can be cultivated on a large scale are considered suitable for use in human nutrition and animal feeds (Bajpai, P. and P. K. Bajpai. 1993. Journal of Biotechnology 30: 161-183). Several single-celled plants, the algae, contain high levels of EPA and DHA and have been considered for the said purposes. References may be made to A. Singh and O.P. Ward (Singh, A. and O.P. Ward, 1997. Advances in Applied Microbiology 45: 272-312). Microorganisms can be easily cultivated on a large scale using cheap nutrients. Several groups of microorganisms contain high amounts of EPA and DHA. Such organisms can be used directly as feed, or the said PUFAs can be extracted from them for further use. Search for microorganisms containing high amounts of DHA and EPA has shown that thraustochytrid protists contain some of the highest amounts of DHA and EPA. Thraustochytrids are already considered of commercial importance. Their cells are used in animal feeds or for extraction of PUFAs for commercial use (Lewis, T.E. et al., 1999, The Biotechnological potential of thraustochytrids. Marine Biotechnology 1: 580-587; US Patent No. 6,451,567 of 17 September 2002).

The Japanese Patent No. 9633263 (1996) describes a strain of a thraustochytrid for application in the food industry such as food-additives, nutritional supplements, as additives for infant milk formula, feedstuffs and drug additives. The strain contains at least 2% of dry wt as DHA. Another Japanese patent No. 980 3671 (1998) describes the production by fermentation of DHA and another PUFA, docosapentaenoic acid (DPA) from lipids of thraustochytrid protists. Cells of thraustochytrid protists may be directly used as feed in aquaculture (US Patent 5,908,622 of 1 June1999). Alternatively, DHA and EPA may be extracted from thraustochytrid cells, using appropriate technlogies (Japanese Patents JP 103105555 of 24.11.1998 and JP 10310556 of 12.5.1997). U.S. Pat. No. 5,340,594 describes a process for production of whole-celled or extracted microbial products using thraustochytrid protists with a high concentration of the omega-3 PUFAs. There is a potential for the use of thraustochytrid protists as human nutraceuticals (Application A428 of Australia New Zealand Food Authority (ANZFA), 12 Dec. 2001). The production of DHA and EPA from thraustochytrid protists requires that they are grown in suitable conditions in fermentors, to yield commercially useful amounts of the two PUFAs. Several research papers and patents address the issue of providing suitable conditions for growth and production of DHA and EPA in thraustochytrids. U.S. Pat. No. 5,340,742 discloses a process for growing the thraustochytrid protists in defined media suitable for their growth. US Patent No. 6,461,839 of 8.10.2002 provides a method of producing PUFAs in *Labyrinthula* sp., using a culture medium containing oil or fatty acid as a carbon source. Yokochi et al., (1999; Applied Microbiology and Biotechnology, 49, 72-76) describe salinity, temperature, carbon source, oil and nitrogen sources for production of high amounts of DGA in the thraustochytrid *Schizochytrium limacinum.* Optimal pH and medium ingredients have also been described for *Thraustochytrium aureum* (Iida T. et al., Journal of Fermentation and Biogengineering, 81: 76-78).

The present invention aims to increase the DHA and EPA levels in thraustochytrid protists independent of ingredients in the culture medium, so that they will provide still higher commercial yields of the said PUFAs. Besides, the above mentioned prior art patents reject a large number of strains, which might have only moderate DHA and EPA concentrations. In the present invention, even strains with moderate amounts of DHA and EPA can be made to produce large amounts of these PUFAs by subjecting them to cold conditions. Bajpai et al. (1991; JAOCS, 68: 509-514) states that the content of PUFA in Thraustochytrium aureum can be increased by growing them at lower temperature (down to 15°C). US Patent No. 5,340,594 states that stressing thraustochytrids with low temperature during growth maintains a high amount of dissolved oxygen in the medium.

However, maintaining large volumes of thraustochytrid culture, amounting to several hundred litres, under cold conditions is an expensive process. Besides, US Patent No. 5,340,594 has makes not reference to the fact that even when the cell biomass has been removed from the aqueous medium containing high oxygen content, but has been stored under cold conditions, their PUFA contents can be enhanced. Further, there is no clue to motivate the Applicants to focus upon variations in the level of the PUFA. Applicants have conducted hundreds of experiments and have developed the method of instant Application. In addition, the said US Patent does not motivate any person skilled in the art to use it for deducing the levels of PUFA. Rather, invention of the instant Application overcomes the above problems by growing cells at ambient temperatures, harvesting the cells and storing them in cold temperature. The DHA and EPA contents increase owing to this storage.

All the above patents relate to screening numerous thraustochytrid cultures, selecting the strain with the highest DHA and EPA content, prepare mutant strains of these and cultivate such strains under optimal culture conditions for commercial production. US Patent No. 6,410,282 provides a method for enhancing polyunsaturated fatty acids, by which the ingredients of the culture medium need not be altered, but by which only the viscosity of the medium is increased by addition of polyvinylpyrrolidone (PVP).

### Objects of the present invention

The main object of the present invention is to enhance the amounts of PUFAs in thraustochytrid protists, which obviate the drawbacks as detailed above.

Another object of the invention is to make strains of thraustochytrids to produce higher amounts of DHA and EPA than they normally produce using optimal nutrient conditions. Yet another object of the present invention is to enhance the levels of these fatty acids by growing the cultures of thraustochytrid protists in a standard medium at ambient temperatures, harvesting the cells and storing them under cold conditions.

Still another object of the present invention is to develop a method of enhancing the levels of Poly-unsaturated fatty acids (PUFA) by 0.5 to 5 times in protists thraustochytrid protists by storing the cells under cold conditions

### Summary of the present invention

The present invention relates to a method of enhancing the levels of Poly-unsaturated fatty acids (PUFA) by 0.5 to 5 times in protists thraustochytrid protists by storing the cells under cold conditions, said method comprising steps of inoculating the protests into a culture medium, growing the culture for 2 to 5 days at room temperature, harvesting the cells by centrifugation to obtain biomass, storing the biomass at about 10°C for time duration ranging between 12 to 48 hours, and obtaining increased PUFA from the stored biomass.

### Detailed description of the present invention

Accordingly, the present invention relates to a method of enhancing the levels of Poly-unsaturated fatty acids (PUFA) by 0.5 to 5 times in protists thraustochytrid protists by storing the cells under cold conditions, said method comprising steps of inoculating the protests into a culture medium, growing the culture for 2 to 5 days at room temperature, harvesting the cells by centrifugation to obtain biomass, storing the biomass at about 10°C for time duration ranging between 12 to 48 hours, and obtaining increased PUFA from the stored biomass.

In an another embodiment of the present invention, wherein a method of enhancing the levels of Poly-unsaturated fatty acids (PUFA) by 0.5 to 5 times in protists thraustochytrid protists by storing the cells under cold conditions, said method comprising steps of:
- inoculating the protests into a culture medium,
- growing the culture for 2 to 5 days at room temperature,
- harvesting the cells by centrifugation to obtain biomass,
- storing the biomass at about 10°C for time duration ranging between 12 to 48 hours, and
- obtaining increased PUFA from the stored biomass.

In another embodiment of the present invention, wherein PUFA is docosahexaenoic acid (DHA), and eicosapentaenoic acid (EHA).

In yet another embodiment of the present invention, wherein using the increased PUFA in animal feeds, human nutrition, nutritional supplement.

In still another embodiment of the present invention, wherein room temperature is ranging between 25 to 30°C.

In still another embodiment of the present invention, wherein thraustochytrid protists are *Thraustochytrium, Schizochytrium,* and *Ulkenia.*

In still another embodiment of the present invention, wherein culture medium comprises peptone of concentration ranging between 0.3 % wt to 3.0% wt., Yeast extract of concentration ranging between 0.005%wt to 0.5% wt, glucose of concentration ranging between 0.005% wt to 5.0% wt, and sea water.

In still another embodiment of the present invention, wherein the concentration of peptones is about 1.5% wt.

In still another embodiment of the present invention, wherein the concentration of Yeast extract is about 0.1 %wt.

In still another embodiment of the present invention, wherein the concentration of glucose is about 1.0% wt.

In still another embodiment of the present invention, wherein even cells containing moderate amount of PUFA under normal conditions produces high quantities.

The present invention provides, by growing the cultures of thraustochytrid protists in a standard medium at ambient temperatures, harvesting the cells and storing them under cold conditions.

Accordingly, the present invention provides a refrigeration method for enhancing levels of polyunsaturated fatty acid levels in thraustochytrid protists and which comprises: (a) providing a thraustochytrid fungus culture belonging to genera such as *Thraustochytrium, Schizochytrium* and *Ulkenia;* (b) culturing cultures of the above protists in a culture medium for 2 to 5 days at a temperature ranging from 25 to 30.degree. C.; (c) harvesting the cells from the above culture by centrifugation; (g) storing the cell biomass under cold temperature conditions for up to 48 hours and (h) of extracting the enhanced amounts of docosahexaenoic acid (DHA) and eicosapentaenoic acids (EPA).

In an embodiment of the present invention, a process is provided to enhance the levels of the PUFAs in cells of thraustochytrid protists.

In yet another embodiment of the present invention, DHA and EPA are enhanced in cells of thraustochytrid protists by growing the cultures in a standard medium, harvesting the cells and storing them under cold conditions.

In yet another embodiment of the present invention, the culture medium used comprising peptone in the range of 0.5% Wt. to 1.5% Wt., preferably, 1.5% Wt.; Yeast extract in the range of 0.01 % Wt. to 0.1% Wt., preferably, 0.1% Wt.; Glucose in the range of 0.01% to 1.0% Wt., preferably, 1.0% Wt.; and Sea water of 100 ml.

In yet another embodiment of the present invention, the length of storage at low temperatures varies from 12 hours to 48 hours.

In yet another embodiment of the present invention, the PUFAs that are enhanced are the DHA and EPA.

The present invention relates to a method for enhancing levels of polyunsaturated fatty acids in thraustochytrid protists. The present invention particularly relates to a process for enhancement of the polyunsaturated fatty acids, docosahexaenoic acid and eicosapentaenoic acid in cells of microorganisms belonging to the group of protists termed thraustochytrids, by storing the cells under cold conditions. The cells thus enriched in the said polyunsaturated fatty acids (PUFAs) can then be utilized more successfully than cells that are not enriched in the PUFAs, in various beneficial applications that require polyunsaturated fatty acids, such as in animal feeds, human nutrition and extraction of the PUFAs for nutritional supplementation.

Accordingly, the present invention provides a method for enhancing levels of polyunsaturated fatty acid levels in thraustochytrid protists, using standard culture media, or media optimized for production of DHA and EPA, and storing the harvested cell biomass in cold temperature. Step a: Providing a culture of a thraustochytrid fungus that contains DHA and EPA; Step b: Inoculating the above said strain in a culture medium; Step c: Growing the culture for 2 days; Step d: Obtaining the cultures for use as inoculum using the above said medium to inoculate a standard medium for culturing thraustochytrids, or a medium optimized for production of EPA and DHA in that particular strain; Step e: Growing the culture separately for 2 to 5 days at a temperature ranging from 25 to 30.degree. C.; Step f: Harvesting the cells from the above culture by centrifugation or other appropriate methods; Step g: Storing the cells at a temperature of 10 C for 12 hours to 48 hours and extracting the enhanced amounts of docosahexaenoic acid (DHA) and eicosapentaenoic acids (EPA) or using the cell biomass for various purposes.

According to the present invention, culture of a candidate species of the thraustochytrid fungus, which contains the omega-3 PUFAs DHA and EPA is first inoculated into a liquid nutrient medium. Strains of protists belonging to other thraustochytrid protists, such as those with the American Type Culture Collection, ATCC Numbers 18906, 18907, 20890, 20891, 20892, 26185 belonging to *Thraustochytrium* sp., No. 28210 belonging to *Thraustochytrium roseum* Gaertner and No. 34304 belonging to *Thraustochytrium aureum* Goldstein may also be used. In addition to thraustochytrids, a member of labyrinthulid protists, which are related to thraustochytrids, such as species of *Labyrinthula* or *Aplanochytrium* may also be used.

A suitable medium for example, is one containing peptone, yeast extract, glucose and sea water. Any other medium that supports good growth of the fungus also may be used. The culture is grown for 2 days at a room temperature ranging from 25 to 30.degree. C. This culture is used as the inoculum and used to inoculate a standard medium as above, or a medium that has been optimized for the production of DHA and EPA for the particular thraustochytrid strain. Cultures may be grown in flasks on a rotary shaker in the laboratory or in a fermentor when large scale cultivation is required. The culture is allowed to grow at room temperature of 25 to 30.degree C or any temperature at which the particular strain grows best.

After a suitable period, for example 2 to 7 days growth, cells from the culture are harvested. This may be done by any appropriate method, such as centrifugation, continuous flow centrifugation, filtration etc. The cell biomass is then stored at a temperature of approximately 10 C for 12 hours to 48 hours. Subsequently, the cells thus stored may be used for all applications that require thraustochytrid cells. Such use may include cell biomass for animal feed, human food supplement or extraction of pure DHA and EPA.

The present invention thus relates to a process to enhance the levels of the omega-3 PUFAs, DHA and EPA. By this process, strains of cultures of thraustochytrids can be made to produce higher levels of these PUFAs than they do under other conditions. Besides, even strains that contain only moderate quantities of these PUFAs under normal conditions can be made to produce greater amounts within their cells. The invention is described in detail hereafter with reference to the accompanying drawings, which are provided merely to illustrate the invention.

The invention is described in detail hereafter with reference to the accompanying drawings which are provided merely to illustrate this invention.

### Brief description of the accompanying drawings

**FIG. 1** represents the DHA contents of a thraustochytrid strain NIOS-1, corresponding in its morphology and life cycle to the genus *Schizochytrium* Goldstein and Belsky, when grown in a liquid nutrient culture medium.
**FIG. 2** represents the EPA contents of a thraustochytrid NIOS-1, corresponding in its morphology and life cycle to the genus *Schizochytrium* Goldstein and Belsky., when grown in a liquid nutrient culture medium.
**FIG.3** represents the DHA contents of a thraustochytrid strain NIOS-2, corresponding in its morphology and life cycle to the species *Thraustochytrium* Sparrow, when grown in a liquid nutrient culture medium.
**FIG. 4** represents the EPA contents of a thraustochytrid NIOS-2, corresponding in its morphology and life cycle to the species *Thraustochytrium* Sparrow., when grown in a liquid nutrient culture medium.
**FIG. 5** represents the DHA contents of a thraustochytrid strain NIOS-3, corresponding in its morphology and life cycle to the species *Ulkenia* Gaertner, when grown in a liquid nutrient culture medium.
**FIG. 6** represents the EPA contents of a thraustochytrid NIOS-3, corresponding in its morphology and life cycle to the species *Ulkenia* Gaertner, when grown in a liquid nutrient culture medium.
**FIG. 7** represents the DHA contents of a thraustochytrid strain NIOS-4, corresponding in its morphology and life cycle to the genus *Ulkenia* Gaertner, when grown in a liquid nutrient culture medium.
**FIG. 8** represents the EPA contents of a thraustochytrid NIOS-1, corresponding in its morphology and life cycle to the genus *Ulkenia* Gaertner, when grown in a liquid nutrient culture medium.
**FIG. 9** represents the DHA contents of a thraustochytrid NIOS-1, corresponding in its morphology and life cycle to the genus *Schizochytrium* sp. when the cell biomass was stored under cold conditions for different lengths of time.
**FIG. 10** represents the EPA contents of a thraustochytrid NIOS-1, corresponding in its morphology and life cycle to the genus *Schizochytrium* sp. when the cell biomass was stored under cold conditions for different lengths of time.

The following examples are given by way of illustrations of the present invention and therefore, should not be construed to limit the scope of the present invention.

### EXAMPLE--1

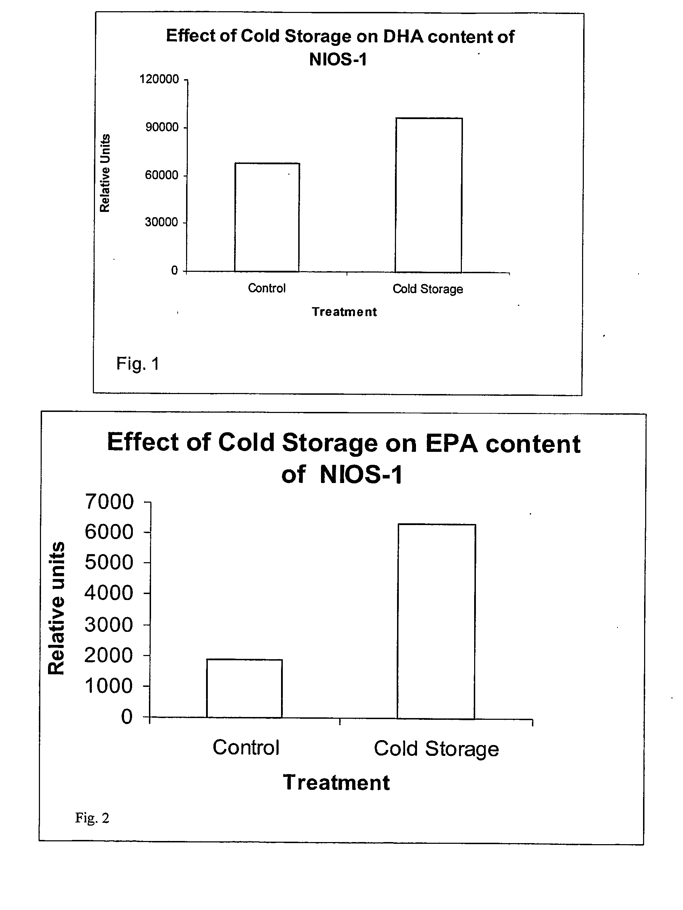

A culture of a thraustochytrid, belonging to strain #NIOS-1, corresponding in its morphology and life cycle to the genus *Schizochytrium* Goldstein and Belsky, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. The cultures were grown for 5 days on a shaker at room temperature of 25-30.degree. C. At the end of this period, cells were harvested by centrifugation. One set of cell biomass was immediately used for fatty acid extraction and analysis by gas chromatography. Another set of the harvested cell biomass was stored in a refrigerator at 10 degrees C for 24 hours, at the end of which fatty acids were extracted and analyzed by gas chromatography. The cell biomass stored in the refrigerator contained nearly 0.5 times more DHA than that not stored in the refrigerator (FIG. 1).

### EXAMPLE-2

A culture of a thraustochytrid, belonging to strain #NIOS-1, corresponding in its morphology and life cycle to the genus *Schizochytrium* Goldstein and Belsky, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. The cultures were grown for 5 days on a shaker at room temperature of 25-30.degree. C. At the end of this period, cells were harvested by centrifugation. One set of cell biomass was immediately used for fatty acid extraction and analysis by gas chromatography. Another set of the harvested cell biomass was stored in a refrigerator at 10 degrees C for 24 hours, at the end of which fatty acids were extracted and analyzed by gas chromatography. The cell biomass stored in the refrigerator contained nearly 3 times more EPA than that not stored in the refrigerator (FIG. 2).

### EXAMPLE-3

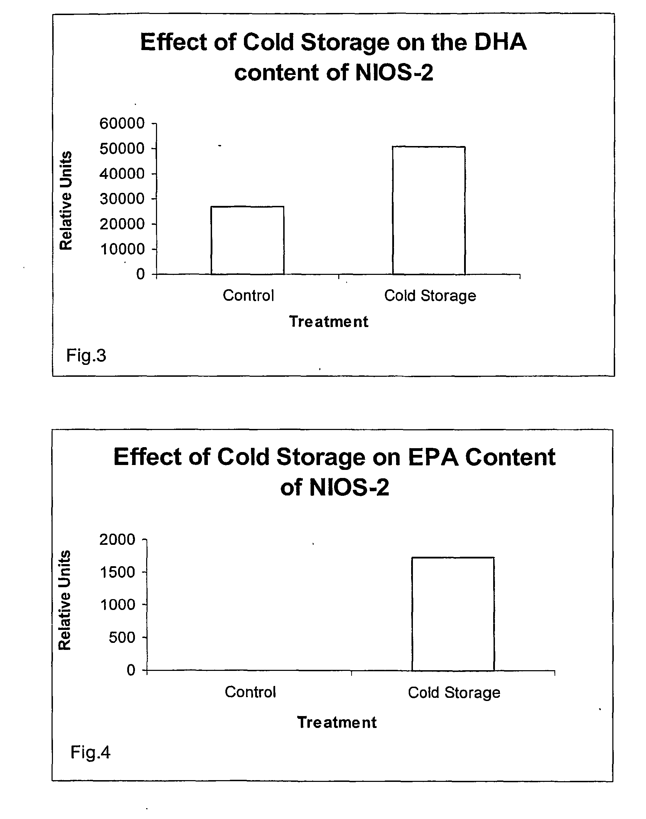

A culture of a thraustochytrid, belonging to strain #NIOS-2, corresponding in its morphology and life cycle to the genus *Thraustochytrium* Gaertner, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. The cultures were grown for 5 days on a shaker at room temperature of 25-30.degree. C. At the end of this period, cells were harvested by centrifugation. One set of cell biomass was immediately used for fatty acid extraction and analysis by gas chromatography. Another set of the harvested cell biomass was stored in a refrigerator at 10 degrees C for 24 hours, at the end of which fatty acids were extracted and analyzed by gas chromatography. The cell biomass stored in the refrigerator contained nearly 2 times more DHA than that not stored in the refrigerator (FIG. 3).

### EXAMPLE-4

A culture of a thraustochytrid, belonging to strain #NIOS-2, corresponding in its morphology and life cycle to the genus *Thraustochytrium* Gaertner, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. The cultures were grown for 5 days on a shaker at room temperature of 25-30.degree. C. At the end of this period, cells were harvested by centrifugation. One set of cell biomass was immediately used for fatty acid extraction and analysis by gas chromatography. Another set of the harvested cell biomass was stored in a refrigerator at 10 degrees C for 24 hours, at the end of which fatty acids were extracted and analyzed by gas chromatography. While the control cultures did not contain any EPA, the cell biomass stored in the refrigerator contained a high amount of this PUFA (FIG. 4).

### EXAMPLE-5

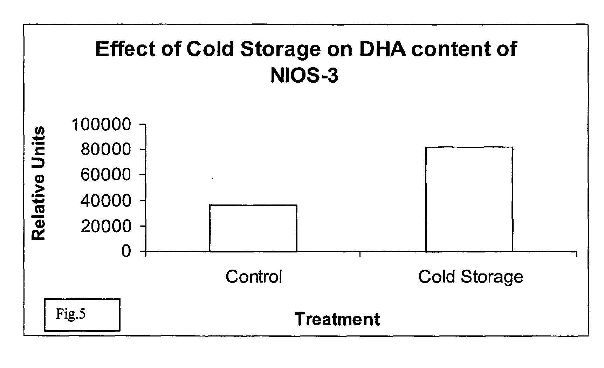

A culture of a thraustochytrid, belonging to strain #NIOS-3, corresponding in its morphology and life cycle to the genus *Ulkenia* Gaertner, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. The cultures were grown for 5 days on a shaker at room temperature of 25-30.degree. C. At the end of this period, cells were harvested by centrifugation. One set of cell biomass was immediately used for fatty acid extraction and analysis by gas chromatography. Another set of the harvested cell biomass was stored in a refrigerator at 10 degrees C for 24 hours, at the end of which fatty acids were extracted and analyzed by gas chromatography. The cell biomass stored in the refrigerator contained nearly 0.5 to 2 times more DHA than that not stored in the refrigerator (FIG. 5).

### EXAMPLE-6

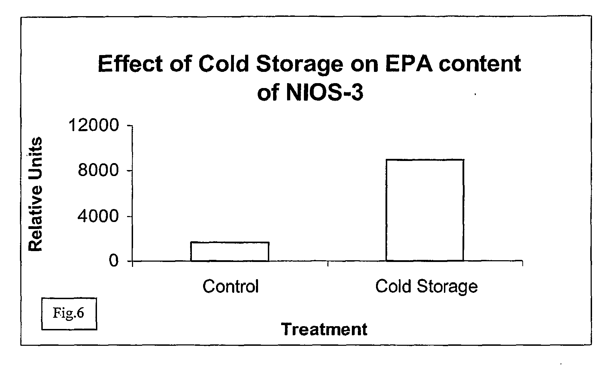

A culture of a thraustochytrid, belonging to strain #NIOS-3, corresponding in its morphology and life cycle to the genus *Ulkenia* Gaertner, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. The cultures were grown for 5 days on a shaker at room temperature of 25-30.degree. C. At the end of this period, cells were harvested by centrifugation. One set of cell biomass was immediately used for fatty acid extraction and analysis by gas chromatography. Another set of the harvested cell biomass was stored in a refrigerator at 10 degrees C for 24 hours, at the end of which fatty acids were extracted and analyzed by gas chromatography. The cell biomass stored in the refrigerator contained nearly 0.5 to 4 times more EPA than that which was not stored in the refrigerator (FIG. 6).

### EXAMPLE-7

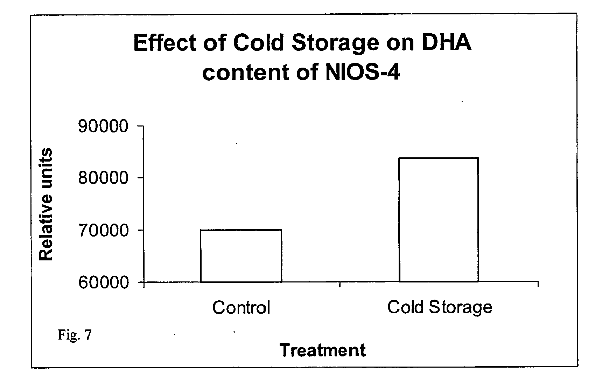

A culture of a thraustochytrid, belonging to strain #NIOS-4, corresponding in its morphology and life cycle to the genus *Ulkenia* Gaertner, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. The cultures were grown for 5 days on a shaker at room temperature of 25-30.degree. C. At the end of this period, cells were harvested by centrifugation. One set of cell biomass was immediately used for fatty acid extraction and analysis by gas chromatography. Another set of the harvested cell biomass was stored in a refrigerator at 10 degrees C for 24 hours, at the end of which fatty acids were extracted and analyzed by gas chromatography. The cell biomass stored in the refrigerator contained nearly 0.5 to 2 times more DHA than that not stored in the refrigerator (FIG. 7).

### EXAMPLE-8

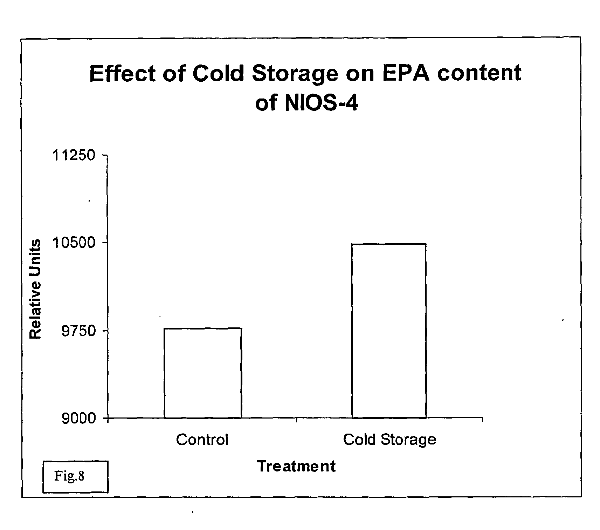

A culture of a thraustochytrid, belonging to strain #NIOS-4, corresponding in its morphology and life cycle to the genus *Ulkenia* Gaertner, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. The cultures were grown for 5 days on a shaker at room temperature of 25-30.degree. C. At the end of this period, cells were harvested by centrifugation. One set of cell biomass was immediately used for fatty acid extraction and analysis by gas chromatography. Another set of the harvested cell biomass was stored in a refrigerator at 10 degrees C for 24 hours, at the end of which fatty acids were extracted and analyzed by gas chromatography. The cell biomass stored in the refrigerator contained nearly 0.5 to 2 times more EPA than that not stored in the refrigerator (FIG. 8).

### EXAMPLE-9

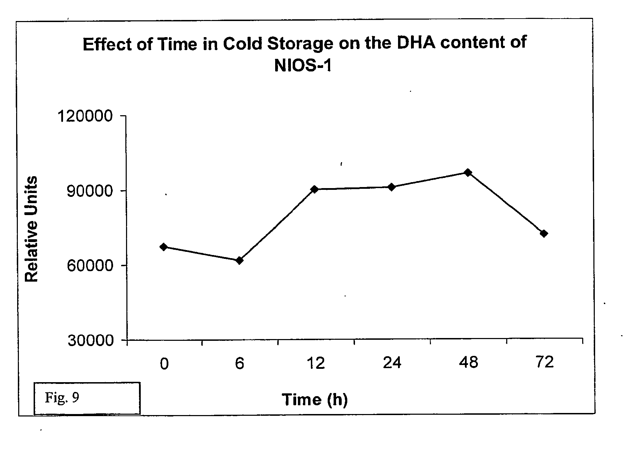

A culture of a thraustochytrid, belonging to strain #NIOS-1, corresponding in its morphology and life cycle to the genus *Schizochytrium* Goldstein and Belsky, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. Six sets of cultures were grown for 5 days on a shaker at room temperature of 25-30.degree. C. At the end of this period, cells were harvested by centrifugation. One set of cell biomass was immediately used for fatty acid extraction and analysis by gas chromatography. The other 5 sets set of harvested cell biomass were stored in a refrigerator at 10 degrees C for 6, 12, 24, 48 and 72 hours respectively, at the end of which fatty acids were extracted and analyzed by gas chromatography. The cell biomass stored in the refrigerator for 6 hours did not show any increase in DHA, that stored for 12-48 hours showed about 50% increase and that stored for 72 hours did not show an increase of DHA (FIG. 9).

### EXAMPLE -10

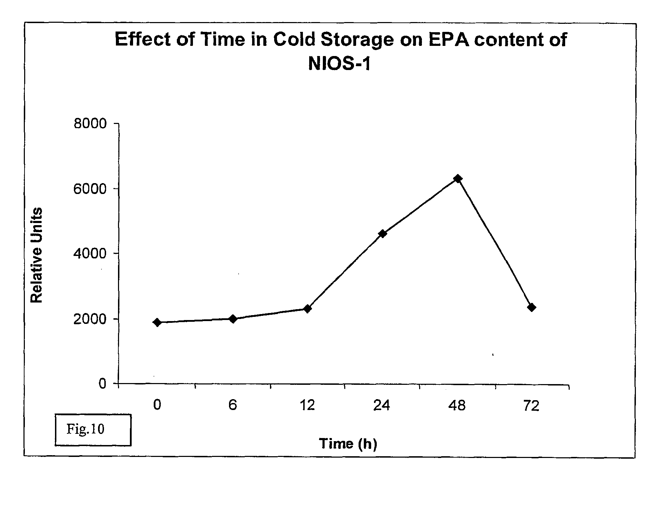

A culture of a thraustochytrid, belonging to strain #NIOS-1, corresponding in its morphology and life cycle to the genus *Schizochytrium* Goldstein and Belsky, was inoculated into 100 ml of a culture medium containing: gelatin peptone--1.5% Wt.; Yeast extract--0.1% Wt.; Glucose--1.0% Wt. and Sea water--100 ml. The cultures were grown for 2 days on a shaker at room temperature of 25-30.degree. C. These cultures were used as inoculum for the experiment. Cultures were set up using a medium with the same composition as above. Six sets of cultures were grown for 5 days on a shaker at room temperature of 25-30.degree. C. At the end of this period, cells were harvested by centrifugation. One set of cell biomass was immediately used for fatty acid extraction and analysis by gas chromatography. The other 5 sets set of harvested cell biomass were stored in a refrigerator at 10 degrees C for 6, 12, 24, 48 and 72 hours respectively, at the end of which fatty acids were extracted and analyzed by gas chromatography. The cell biomass stored in the refrigerator for 6 hours did not show any increase in DHA, that stored for 12 hours revealed a marginal increase of DHA, that stored at 24 hours showed an increase of nearly 100 %, that stored for 48 hours showed an increase of nearly 300 % and that stored for 72 hours did not show an increase of epa (FIG. 10).

**The main advantages of the present invention are:**
1. The DHA and EPA levels of thraustochytrids normally present in cultures can be further enhanced using a simple technique.
2. Even those strains that have only moderate quantities of DHA and EPA can be enriched in these fatty acids.
3. Increase in DHA and EPA levels is effected by storing the cell biomass in cold, for a period of 12 to 48 hours.

## Claims

1. A method of enhancing the levels of Poly-unsaturated fatty acids (PUFA) by 0.5 to 5 times in thraustochytrid protists by storing the cells under cold conditions, said method comprising steps of:
a. inoculating the protists into a culture medium,
b. growing the culture for 2 to 5 days at room temperature,
c. harvesting the cells by centrifugation to obtain biomass,
d. storing the biomass at about 10°C for time duration ranging between 12 to 48 hours, and
e. obtaining increased PUFA from the stored biomass.

2. A method as claimed in claim 1, wherein PUFA is docosahexaenoic acid (DHA), and eicosapentaenoic acid (EHA).

3. A method as claimed in claim 1, wherein using the increased PUFA in animal feeds, human nutrition, nutritional supplement.

4. A method as claimed in claim 1, wherein room temperature is ranging between 25 to 30°C.

5. A method as claimed in claim 1, wherein thraustochytrid protists are *Thraustochytrium, Schizochytrium,* and *Ulkenia.*

6. A method as claimed in claim 1, wherein culture medium comprises peptone of concentration ranging between 0.3 % wt to 3.0% wt., Yeast extract of concentration ranging between 0.005%wt to 0.5% wt, glucose of concentration ranging between 0.005% wt to 5.0% wt, and sea water.

7. A method as claimed in claim 6, wherein the concentration of peptones is about 1.5% wt.

8. A method as claimed in claim 6, wherein the concentration of Yeast extract is about 0.1 %wt.

9. A method as claimed in claim 6, wherein the concentration of glucose is about 1.0% wt.

10. A method as claimed in claim 1, wherein even cells containing moderate amount of PUFA under normal conditions produces high quantities.

## Patentansprüche

1. Verfahren zum Erhöhen der Konzentration von mehrfach ungesättigten Fettsäuren (PUFA) um 0,5- bis 5-mal in Thraustrochytrid-Protisten mittels Lagerung der Zellen unter kalten Bedingungen, wobei das Verfahren die Schritte umfaßt:
a. Inokulieren der Protisten in ein Kulturmedium,
b. Wachsenlassen der Kultur für 2 bis 5 Tage bei Zimmertemperatur,
c. Ernten der Zellen mittels Zentrifugation, um Biomasse zu erhalten,
d. Lagern der Biomasse bei ungefähr 10°C für eine Zeitdauer im Bereich zwischen 12 und 48 Stunden,
e. Erhalten von vermehrten PUFA aus der gelagerten Biomasse.

2. Verfahren nach Anspruch 1, wobei die PUFA Docosahexaenoic-Säure (DHA) und Eicosapentaenoic-Säure (EHA) ist.

3. Verfahren nach Anspruch 1, wobei die vermehrten PUFA in Tierfutter, menschlicher Nahrung und Nahrungszusatzstoffen verwendet werden.

4. Verfahren nach Anspruch 1, wobei die Zimmertemperatur im Bereich zwischen 25 bis 30°C liegt.

5. Verfahren nach Anspruch 1, wobei die Thraustochytrid-Protisten *Thraustochytrium, Schizochytrium* und *Ulkenia* sind.

6. Verfahren nach Anspruch 1, wobei das Kulturmedium Pepton in einer Konzentration im Bereich zwischen 0,3 Gew.% bis 3,0 Gew.%, Hefeextrakt in einer Konzentration im Bereich zwischen 0,005 Gew.% bis 0,5 Gew.%, Glukose in einer Konzentration im Bereich zwischen 0,005 Gew.% bis 5,0 Gew.% und Meerwasser umfaßt.

7. Verfahren nach Anspruch 6, wobei die Konzentration der Peptone ungefähr 1,5 Gew.% beträgt.

8. Verfahren nach Anspruch 6, wobei die Konzentration des Hefeextrakts ungefähr 0,1 Gew.% beträgt.

9. Verfahren nach Anspruch 6, wobei die Konzentration von Glukose ungefähr 1,0 Gew.% beträgt.

10. Verfahren nach Anspruch 1, wobei sogar Zellen, die mäßige Mengen an PUFA unter normalen Bedingungen enthalten, große Mengen produzieren.

## Revendications

1. Procédé permettant d'accroître les taux d'acides gras polyinsaturés (PUFA) de 0,5 à 5 fois dans des protistes du type thraustochitrium en stockant les cellules en conditions de froid, ledit procédé comprenant les étapes de:
a) inoculer les protistes dans un milieu de culture,
b) accroître la culture pour 2 à 5 jours à la température de la chambre,
c) récolter les cellules par centrifugation pour obtenir la biomasse,
d) stocker la biomasse à environ 10 °C pour une durée de temps située entre 12 à 48 heures, et
e) obtenir les PUFA accrûs de la biomasse stockée.

2. Procédé selon la revendication 1, où le PUFA est l'acide docosahexaénoïque (DHA) et l'acide eicosapentaénoïque (EHA).

3. Procédé selon la revendication 1, où en utilisant le PUFA accrû dans la nourriture des animaux, la nutrition humaine, le supplément nutritionnel.

4. Procédé selon la revendication 1, où la température de la chambre est située entre 25 à 30 °C.

5. Procédé selon la revendication 1, où les protistes de thraustochitrium sont Thraustochitrium, Schizochitrium, et Ulkenia.

6. Procédé selon la revendication 1, où le milieu de culture comprend la peptone de concentration située entre 0,3 % en poids à 3,0 % en poids, l'extrait de levure de concentration située entre 0,005 % en poids à 0,5 % en poids, le glucose de concentration située entre 0,005 % en poids à 5,0 % en poids, et l'eau de mer.

7. Procédé selon la revendication 6, où la concentration de peptones est environ 1,5 % en poids.

8. Procédé selon la revendication 6, où la concentration d'extrait de levure est environ 0,1 % en poids.

9. Procédé selon la revendication 6, où la concentration de glucose est environ 1,0 % en poids.

10. Procédé selon la revendication 1, où même les cellules contenant des quantités modérées de PUFA sous les conditions normales produisent des grandes quantités.
